# EUROPEAN PATENT SPECIFICATION

(11) **EP 3 355 805 B1**
(45) Date of publication and mention of the grant of the patent: **12.08.2020**
(21) Application number: 16850493.4
(22) Date of filing: 29.09.2016
(51) Int. Cl.: A61B 17/068, A61B 17/072, A61B 17/11

(54) **LINEAR STAPLER COMPRISING A MECHANISM OF INTERMEDIATE CLOSURE**
MEHRKLAMMERNAHTGERÄTS MIT EINEM MECHANISMUS FÜR EIN TEILWEISES SCHLIESSEN
AGRAFEUSE LINÉAIRE AVEC MÉCANISME DE FERMETURE INTERMÉDIAIRE

(30) Priority: 30.09.2015 PL 41423215
(43) Date of publication of application: 08.08.2018
(73) Proprietor: "Konmex" Spólka Z Ograniczona Odpowiedzialnoscia, 05-804 Pruszków (PL)
(72) Inventor: BRODACZEWSKI, Wieslaw, 05-410 Józefów (PL); DECEWICZ, Andrzej, Nottingham NG3 4FQ (GB)
(74) Representative: Kondrat, Mariusz
(86) International application number: PCT/IB2016/055820
(87) International publication number: WO 2017/056028

(56) References cited:
- EP-A2- 0 136 950
- WO-A1-2013/022703
- CN-U- 203 182 959
- US-A- 4 633 874
- US-A- 5 605 272
- US-A1- 2012 312 861

## Description

The object of the invention is a linear surgical stapler comprising a mechanism of intermediate closure. .

A document US4429695 discloses a linear stapler for a load with a knife which comprises two cooperating frames, the upper one and a lower one with the jaws, clamps, pushers, cutting unit, locking handle and two double rows of staples. A surgical device is equipped with two cooperating frames provided with longitudinal jaws, wherein one of the jaws is adapted to successively activate staples which are formed by grooves of the anvil located on a second, opposite jaw. Moreover, this device has a laterally spaced pushers supporting the jaws, wherein each is characterized by a knife handle, which is placed between said jaws. Moreover, this device has a blocking handle mounted on the pin, which determines whether the device is half- opened or closed. The handle operates through the grooves located thereon, which cooperate with a pin placed on the lower and upper frame.

A description of the document AU615131 dicloses a device which comprises two parallel pairs of jaws, one of which contains the staples, a second jaw the grooves that form the staples. The device is characterized in that the upper jaw has a pin that rotatably passes through a channel in the lower jaw, thus connecting the two jaws in one piece with the third element, with the pusher and knife attached thereto. The device is blocked prior to use by a second rotation pin located on the lower jaw, which allows the movement of the joint switch with a grip part, which closes the device and allows to fire it.

A document US8365971 discloses a surgical stapling device which comprises a first handle, an anvil, a staples cartridge mounted on a second handle and a cutting unit. The device is characterized in that the anvil is detachably connected with the staples cartridge in such a way that they can be completely separated. Combining the staples cartridge located in the second handle takes place through the engagement of a recess located in a fork attached to the first handle to the rotatable element attached to the second handle, thereby closing the surgical device and allowing firing it.

The devices known in the prior art consist of two opposing jaws, wherein surgical staples are located on one of them, which are activated after firing the load, then shaped by the grooves on the other jaw in the anvil and closed on the tissue. The system activating the staples consists of a cutting unit and a pusher, which consists of staple pushers, a knife pusher and a knife. Known devices have demountable parts, which have to be put together before use. Putting together takes place by means of pins located on one element and the channels located on the second element. Surgical devices known in the prior art have two working positions, open, wherein the device is put together, without the stability of contributory elements and closed, in which load firing and tissue stapling and/anastomosis takes place. Unfortunately, known solutions have some disadvantages. The biggest disadvantage of using linear staplers for a load with a knife is the inability to adjust the position of intermediate closure of the stapler and thus the inability to adjust the settings of the tissue relative to the jaws of the stapler before stapling/anastomosis. The prior art works on the devices which have two working positions, i.e. fully open, in which the stapler is dismantled and the load is replaced as well as fully closed, in which the stapling takes place. In the solutions on the market, a state of partial closure of the stapler means a condition when the jaws are inert and free and can move relative to each other, and their stabilization before the complete closure can only be done through the use of other tools.

US4633874 discloses a surgical stapling instrument, comprising: first and second cooperating elongate jaw members, one of said jaw members including staple carrying means adapted to receive a plurality of staples arranged in at least one row, and said other jaw member including anvil means adapted to form said staples, pusher means for driving the staples from said staple carrying means into tissue gripped between said jaw members and forming the staples against said anvil means to produce at least one row of staples in the tissue, means for latching said jaw members together with the tissue located between said staple carrying means and said anvil means, jaw clamping means for applying clamping forces to said jaw members to urge said staple carrying means and said anvil means together during the formation of said staples,and means for retaining said latching means in a partially latched position with said jaw members loosely connected together to permit said jaw members to be moved apart and said staple carrying means and said anvil means to be adjusted in position on the tissue without disconnecting said jaw members from each other.

The aim of the invention was to provide a mechanism for intermediate closure of the linear stapler for a load with a knife, which allows the user to safely manipulate tissue in an intermediate position before the final closing of the stapler and firing a load.

A solution of the invention is a new solution that is different from the solutions currently on the market. The mechanism for intermediate closure of the linear stapler for a load with a knife increases comfort during surgery and clarifies tissues stapling. A surgical device in an intermediate closure position is stable, protects the stapler front of a wider opening or the jaws closing. The position allows precise positioning of tissue on the load, which increases the precision of stapling.

The solution of the invention consists in that the a ratchet bed system is on the anvil, which consists of the ratchet seat, which includes a ratchet bed and ratchet recess, which when closing the pressing cover meets the ratchet spring and the ratchet itself, which slides over the top part of the ratchet seat, and then falls/slides into the ratchet bed and makes it impossible to return to the open state of the stapler. In the intermediate closure position the stapler is stable and allows to easily manipulate tissue located between the load and the upper jaw/anvil of the stapler so as to increase the stapling precision, before the final closing of the stapler and stapling/anastomosis.

The essence of the invention is a linear surgical stapler comprising: an anvil, a load bed configured to receive a staple cartridge, the load bed being pivotably connected to the anvil, a pressing cover connected to the load bed and the anvil such that pressing the pressing cover towards the anvil approximates the anvil towards the load bed. The stapler is characterized by the anvil further comprising a ratchet bed system, which includes a ratchet seat, a ratchet bed and a ratchet recess, and the pressing cover comprising a ratchet spring with a ratchet, wherein the stapler is configured such that wherein when pressing the pressing cover towards the anvil, the ratchet slides over the outer surface of the ratchet seat, which at an acute angle bends in a recess being the ratchet bed, until it enters the ratchet bed and the inner surface of the ratchet contacts the surface of the ratchet bed, the intermediate closure position being defined and retained by the ratchet contacting the ratchet seat and the ratchet bed, and further characterized by the stapler being configured such that after further pressing the pressing cover, when the pressing cover is fully pressed against the anvil, the ratchet is located under the ratchet seat in the area between the ratchet seat and the ratchet recess.

Preferably, the ratchet seat has a form of an irregular quadrilateral figure, whose upper part is convex and the ratchet bed is arranged parallel to the ratchet recess.

In other words the invention relates to a mechanism for intermediate closure of the stapler for a load with a knife for fixing tissue fragments, which consists of the anvil comprising a ratchet bed system, which consists of a ratchet seat with a ratchet bed and of the anvil absorber, an upper jaw, a stabilizing groove and the anvil pin through which the anvil is permanently, but rotatably connected to the pressing cover at left side of which the ratchet spring and the ratchet is located.

The linear stapler for a load with a knife is used in general surgery, in abdominal surgery, thoracic surgery, gynecology and in pediatric surgery, used to resection or transsection of organs and tissues. In the case of reusable staplers, before the first use the stapler must be washed from the residue of a preservative and sterilized in an autoclave. After each use, stapler should be cleaned and sterilized, so that it can be used again. It refers only to the reusable device, since stapler for a load with a knife can also be disposable and in that case is supplied in a sterile state. A disposable stapler is delivered without a load, due to the fact that it can be replaced several times during one surgical procedure, after which the stapler is disposed. Both the loads for a reusable stapler as well as the disposable one are disposable and are supplied in separate containers in a sterile state. The load is placed/inserted into both the reusable and disposable stapler in the load bed, which in turn connects to an anvil with a stabilizing socket.

The linear stapler for a load with a knife is used by putting it together in one piece with the load. Putting together is done by inserting the load in the load bed, followed by insertion of the stabilizing groove located on the anvil in the stabilizing socket located in the load bed. Thereafter the load bed pin enters the channel of the load bed by pivoting a pressing cover. In order to achieve the position of the intermediate closure of the stapler for a load with a knife the pressing cover should be pressed against the anvil so that the ratchet sliding over the top surface of the ratchet seat slides so deeply until all of the ratchet enters the ratchet bed and abut the surface of the inner ratchet with the surface of the ratchet bed.

In this position the stapler is stable, there is enough space between the upper jaw and the cartridge where tissues can be freely manipulated for optimal alignment before stapling/anastomosis. In this position there is no danger of stapler closing or opening.

A mechanism for intermediate closure of the linear stapler for a load with a knife consists of the anvil on which the ratchet seat is located, which includes the ratchet bed, then the recess, the anvil pin and load bed pin, the pressing cover which comprises a ratchet spring with a ratchet.

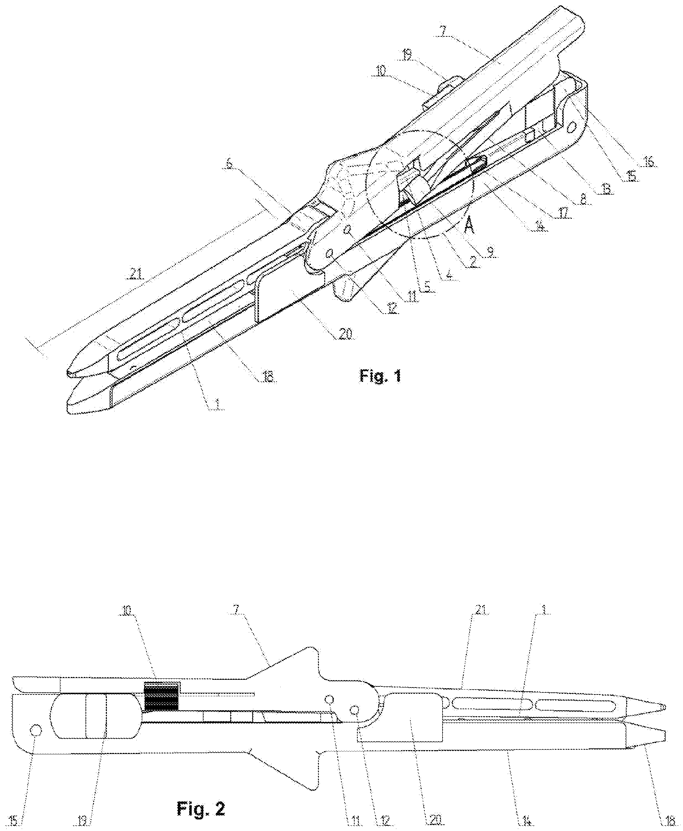

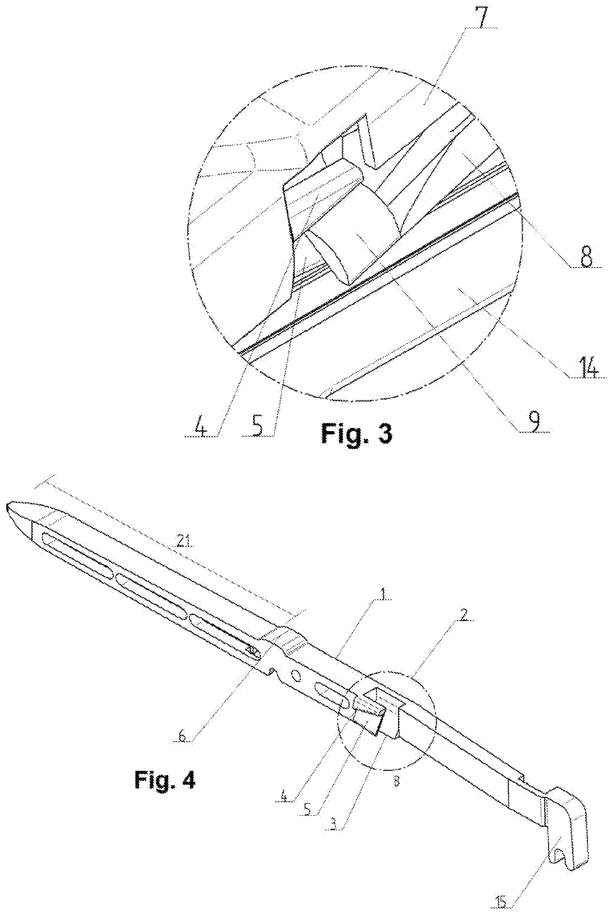

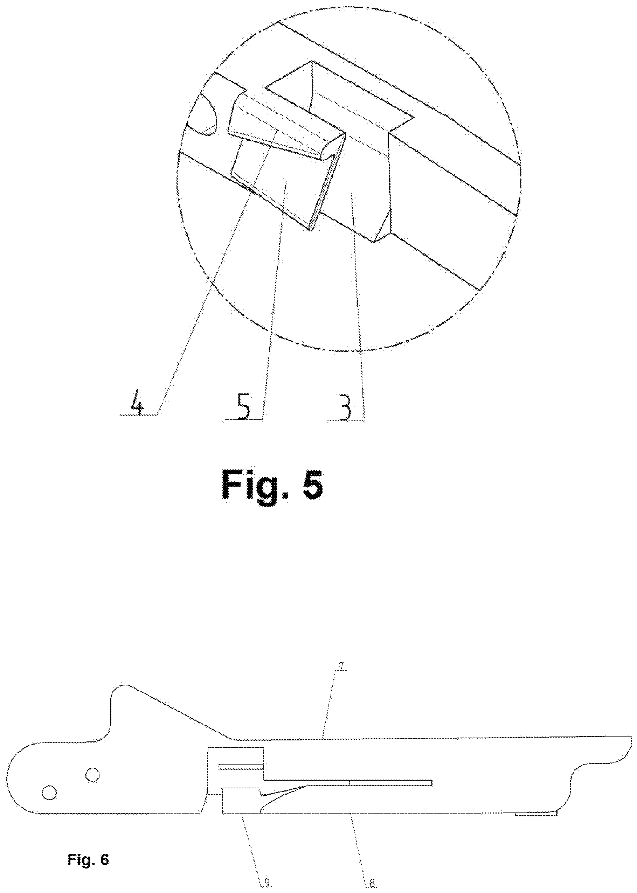

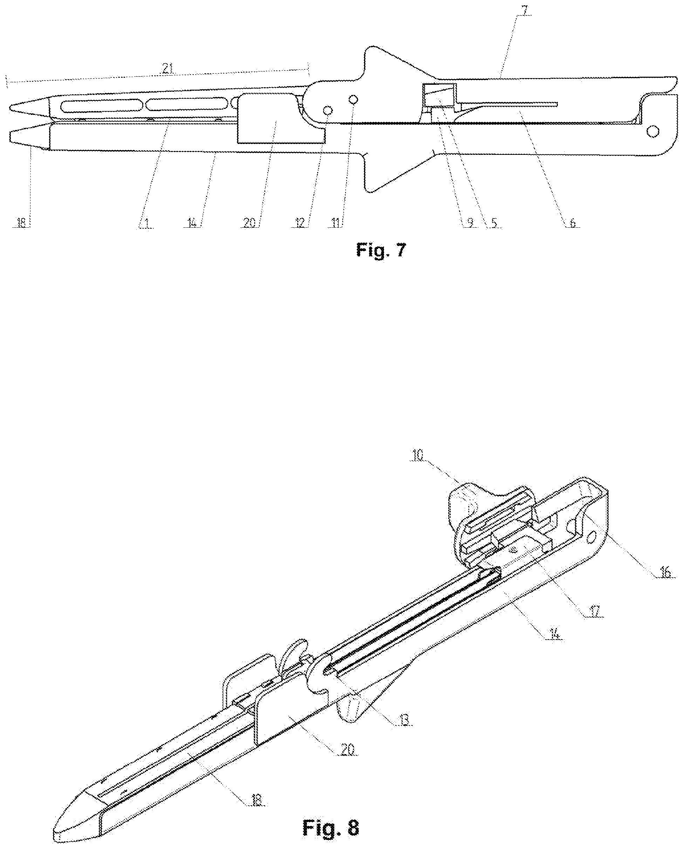

The object of the invention in the embodiment was illustrated in the drawing, in which Fig. 1 shows an isometric view of the entire left side of the intermediately closed linear stapler for a load with a knife with a marked detail field A, i.e. a mechanism for intermediate closure of the linear stapler for a load with a knife, Fig. 2 shows a perspective view of the right side of the closed linear stapler for a load with a knife, Fig. 3 shows an enlargement of detail A i.e. isometric view of the mechanism for intermediate closure of the linear stapler for a load with a knife, Fig. 4 is an isometric view of the anvil from the left side with a marked detail field B i.e. a ratchet bed system, fig. 5 shows an enlargement of detail B i.e. a ratchet bed system with an indication of the ratchet system elements, fig. 6 shows a perspective view of a pressing cover from the left side. Fig. 7 shows a perspective view of a linear stapler for a load with a knife in a closed position, fig. 8 shows an isometric view of the load bed with a load.

A linear stapler for a load with a knife comprises four main elements: an anvil 1, a pressing cover 7, a load 17 and a load bed 14, wherein a load 17, a load bed 14 and the anvil 1 connected permanently but pivotally connected to the pressing cover 7 may be decomposed into three separate parts.

A mechanism for intermediate closure of the linear stapler consists of an anvil 1 of an irregular, elongated, narrow shape, flattened at one end and with a protruding perpendicular stabilizing groove 15 at the other end, with a notch at the height of the upper jaw which houses a ratchet bed system 2 in the central part, which consists of a ratchet recess 3 and a ratchet seat 4 in the form of the irregular quadrilateral figure, whose upper part is convex and at an acute angle bends in the recess being the ratchet bed 5. The plate-shaped ratchet bed 5 is located within the ratchet recess and forms a surface parallel relative to the ratchet recess 3. Using an anvil pin 11 , the anvil 1 connects to a pressing cover 7 with a ratchet spring 8 with a rectangular ratchet 9 on the left side of the pressing cover 7, which on the outer side has a convex surface and the flat surface at the inner side. On the left side of the pressing cover there is a latch 10 button. In turn, the anvil 1 connects with the load 14 in its distal part, by the stabilizing groove 15, which enters the stabilizing socket 16.

The load 17 which is located between the anvil 1 and the load bed 14, in which it is inserted, is finished on one side with a fixedly mounted staples cartridge 18, on the other side there is a handle of the pusher 19.

A stabilizing socket 16, a load bed channel 13 are in the load bed 14, and the knife guard 20 is on the outer side.

Due to the fact that three main elements of the linear stapler for a load with a knife can be decomposed into three separate parts, in order to use the stapler it should be put together. Putting together is done by inserting the load 17 in the load bed 14. Then, the pressing cover 7 which is fixedly, but pivotally connected to the anvil 1 using an anvil pin 11, should be pivoted at the greatest angle to the anvil 1, the angle at which the resistance occurs caused by contact of the absorber of the anvil 6 is located on the back of the anvil 1 between the upper jaw 21 and the anvil pin 11 with the pressing cover 7.

After pivoting the pressing cover 7, the stabilizing groove 15 located on the rear part of the anvil 1 enters the stabilizing socket 16 in the rear part of the load bed 14. The load bed pin 12 enters into hooked recess of the bed load channel 13.

A mechanism for intermediate closure is activated in the course of pressing the pivoted pressing cover 7 towards the anvil 1. This causes the inner surface of the ratchet 9 to slide over the upper surface of the ratchet 4, then the entire ratchet 9 enters into the ratchet bed 5 and the inner surface 9 of the ratchet contacts the surface of the ratchet bed 5.

In this position the stapler is stable, there is enough space between the upper jaw 21 and the staples cartridge 18, where tissues can be freely manipulated for optimal alignment before stapling/anastomosis in which one can freely manipulate tissue and there is no danger of stapler closing or opening.

Then in order to close the stapler and perform the stapling/anastomosis, the pressing cover 7 should be pressed against the anvil 1, then the entire ratchet 9 slides under the ratchet bed 5, into the free space between the ratchet seat 4 and the ratchet recess 3. Only in this position tissue stapling/anastomosis can be performed by moving the pusher handle 19 along the load bed 14.

## Claims

1. A linear surgical stapler comprising:
an anvil,
a load bed configured to receive a staple cartridge, the load bed being pivotably connected to the anvil,
a pressing cover connected to the load bed and the anvil such that pressing the pressing cover towards the anvil approximates the anvil towards the load bed,
**characterized by** the anvil further comprising a ratchet bed system (2), which includes a ratchet seat (4), a ratchet bed (5) and a ratchet recess (3), and the pressing cover (7) comprising a ratchet spring (8) with a ratchet (9), wherein the stapler is configured such that when pressing the pressing cover (7) towards the anvil (1), the ratchet (9) slides over the outer surface of the ratchet seat (4), which at an acute angle bends in a plate-shaped recess being the ratchet bed (5), until the ratchet (9) enters the ratchet bed (5) and the inner surface of the ratchet (9) contacts the surface of the ratchet bed (5), the intermediate closure position being defined and retained by the ratchet (9) contacting the ratchet seat (4) and the ratchet bed (5),
and
**further characterized by** the stapler being configured such that after further pressing the pressing cover (7) in the intermediate closure position towards the anvil (1), when the pressing cover (7) is fully pressed against the anvil (1), the ratchet (9) is located under the ratchet seat (4) in the area between the ratchet seat (4) and the ratchet recess (3).

2. The stapler according to claim 1 **characterized in that** the ratchet seat (4) has a form of an irregular quadrilateral figure, whose upper part is convex and the ratchet bed (5) is arranged parallel to the ratchet recess (3).

## Patentansprüche

1. Ein lineares chirurgisches Klammergerät, bestehend aus:
einem Amboss,
einem Klammermagazin, das zur Aufnahme einer Klammerpatrone bestimmt ist,
wobei das Klammermagazin mit dem Amboss durch eine Druckplatte schwenkbar verbunden ist, die so an das Klammermagazin und den Amboss angebracht ist. Durch das Pressen der Druckplatte zum Amboss hin nähert sich der Amboss dem Klammermagazin an,
**und dadurch gekennzeichnet ist, dass** der Amboss ferner eine Aufnahmevorrichtung (2) umfasst, die aus einem Schiebersitz (4), einer Schiebestange (5) und einer Aussparung (3) sowie einer Andruckplatte (7) einschließlich einer Feder (8) mit einer Sperrklinke (9) besteht, wobei das Klammergerät so konfiguriert ist, dass beim Pressen der Andruckplatte (7) in Richtung des Ambosses (1) die Sperrklinke (9) über die Außenfläche des Schiebersitzes (4) gleitet, die sich in einer flächenförmigen Aussparung der Schiebestange (5) biegt bis die Innenfläche der Sperrklinke (9) die Oberfläche der Schiebestange (5) berührt und die Sperrklinke (9) in der Schiebestange (5) gleitet, wobei die Zwischenschließposition durch die Sperrklinke (9), die den Schiebersitz (4) und die Schiebestange (5) berührt, definiert und gehalten wird,
und **weiter dadurch gekennzeichnet** wird, dass das Klammergerät so konfiguriert ist, dass nach weiterem Drücken der Andruckplatte (7) in der Zwischenverschließposition gegen den Amboss (1), wenn die Andruckplatte (7) vollständig gegen den Amboss (1) gedrückt wurde, sich die Sperrklinke (9) unter dem Schiebersitz (4) im Bereich zwischen dem Schiebersitz (4) und der Aussparung (3) befindet.

2. Das Klammergerät nach Anspruch 1, **dadurch gekennzeichnet, dass** der Schiebersitz (4) die Form einer unregelmäßigen viereckigen Figur hat, deren oberer Teil konvex ist und deren Schiebestange (5) parallel zur Aussparung (3) verläuft.

## Revendications

1. Une agrafeuse chirurgicale linéaire comprenant :
une enclume,
un lit de chargement configuré pour recevoir une cartouche d'agrafes, le lit de chargement étant relié de manière pivotante à l'enclume,
un couvercle de pressage relié au lit de chargement et à l'enclume de telle sorte que le fait de presser le couvercle de pressage vers l'enclume rapproche l'enclume du lit de chargement,
**caractérisée en ce que** l'enclume comprend en outre un système de lit à cliquet (2), qui comprend un siège à cliquet (4), un lit à cliquet (5) et un évidement à cliquet (3), et **en ce que** le couvercle de pressage (7) comprend un ressort à cliquet (8) avec un cliquet (9), dans lequel l'agrafeuse est configurée de telle sorte que, lorsque l'on presse le couvercle de pressage (7) vers l'enclume (1), le cliquet (9) glisse sur la surface extérieure du siège à cliquet (4), qui se courbe à un angle aigu dans un évidement en forme de plaque constituant le lit à cliquet (5), jusqu'à ce que le cliquet (9) entre dans le lit à cliquet (5) et que la surface intérieure du cliquet (9) entre en contact avec la surface du lit à cliquet (5), la position de fermeture intermédiaire étant définie et maintenue par le contact du cliquet (9) avec le siège à cliquet (4) et le lit à cliquet (5), et
**caractérisée en outre par le fait que** l'agrafeuse est configurée de telle sorte qu'après avoir pressé davantage le couvercle de pression (7) dans la position de fermeture intermédiaire vers l'enclume (1), lorsque le couvercle de pression (7) est entièrement pressé contre l'enclume (1), le cliquet (9) est situé sous le siège à cliquet (4) dans la zone entre le siège à cliquet (4) et l'évidement à cliquet (3).

2. L'agrafeuse selon la revendication 1 **caractérisée en ce que** le siège à cliquet (4) a la forme d'une figure quadrilatérale irrégulière, dont la partie supérieure est convexe et le lit à cliquet (5) est disposé parallèlement à l'évidement à cliquet (3).
